# EUROPEAN PATENT APPLICATION

(11) **EP 1 676 923 A1**
(43) Date of publication of application: **05.07.2006**
(21) Application number: 04772559.3
(22) Date of filing: 25.08.2004
(51) Int. Cl.: C12P 19/28, A61K 38/47, A61P 19/04, A61P 25/00, A61P 43/00, C12N 9/42

(54) **SUGAR CHAIN-CUTTING AGENT**

(30) Priority: 26.08.2003 JP 2003301943
(71) Applicant: SEIKAGAKU CORPORATION, Chiyoda-ku, Tokyo 100-0005 (JP)
(72) Inventor: NARIMATSU, H., Nat. Inst. Adv. Ind. Sce & Tech., Tsukuba-shi, Ibara (JP); KIMATA, Koji (Room 1201, Esupoa-Yashiroguchi), Nagoya-shi, Aichi 465-0013 (JP); YADA, Toshikazu, Kuwana-shi, Mie 511-0056 (JP)
(74) Representative: HOFFMANN EITLE
(86) International application number: PCT/JP2004/012603
(87) International publication number: WO 2005/019465

(57) **Abstract**

A sugar chain-cleaving agent, which comprises a protein having activity of degrading chondroitin and/or chondroitin sulfate; a medicament which comprises the sugar chain-cleaving agent as an active ingredient; a method for specifically cleaving a sugar chain, which comprises reacting the protein with chondroitin and/or chondroitin sulfate; and a method for specifically producing a sugar chain of which molecular weight is decreased, which reacting the protein with chondroitin and/or chondroitin sulfate.

SEQUENCE LISTING

<110> NATIONAL INSTITUTE OF ADVANCED INDUSTRIAL SCIENCE AND TECHNOLOGY

<110> SEIKAGAKU CORPORATION

<120> Glyco-degradation reagent

<130> P05001700

<150> JP 2003-301943
<151> 2003-08-26

<160> 2

<170> PatentIn Ver. 3.1

<210> 1
<211> 1746
<212> DNA
<213> Homo sapiens

<220>
<221> CDS
<222> (25)..(1623)

<400> 1
tctggacgca tggctgattc ctga atg atg atg gtt cgc cgg ggg ctg ctt     51
                           Met Met Met Val Arg Arg Gly Leu Leu
                           1               5

gcg tgg att tcc cgg gtg gtg gtt ttg ctg gtg ctc ctc tgc tgt gct     99
Ala Trp Ile Ser Arg Val Val Val Leu Leu Val Leu Leu Cys Cys Ala
10                  15                  20                  25

atc tct gtc ctg tac atg ttg gcc tgc acc cca aaa ggt gac gag gag     147
Ile Ser Val Leu Tyr Met Leu Ala Cys Thr Pro Lys Gly Asp Glu Glu
                30                  35                  40

cag ctg gca ctg ccc agg gcc aac agc ccc acg ggg aag gag ggg tac     195
Gln Leu Ala Leu Pro Arg Ala Asn Ser Pro Thr Gly Lys Glu Gly Tyr
            45                  50                  55

cag gcc gtc ctt cag gag tgg gag gag cag cac cgc aac tac gtg agc     243
Gln Ala Val Leu Gln Glu Trp Glu Glu Gln His Arg Asn Tyr Val Ser
        60                  65                  70

agc ctg aag cgg cag atc gca cag ctc aag gag gag ctg cag gag agg     291
Ser Leu Lys Arg Gln Ile Ala Gln Leu Lys Glu Glu Leu Gln Glu Arg
    75                  80                  85

agt gag cag ctc agg aat ggg cag tac caa gcc agc gat gct gct ggc     339
Ser Glu Gln Leu Arg Asn Gly Gln Tyr Gln Ala Ser Asp Ala Ala Gly
90                  95                  100                 105

ctg ggt ctg gac agg agc ccc cca gag aaa acc cag gcc gac ctc ctg 387
Leu Gly Leu Asp Arg Ser Pro Pro Glu Lys Thr Gln Ala Asp Leu Leu
                110                 115                 120

gcc ttc ctg cac tcg cag gtg gac aag gca gag gtg aat gct ggc gtc 435
Ala Phe Leu His Ser Gln Val Asp Lys Ala Glu Val Asn Ala Gly Val
            125                 130                 135

aag ctg gcc aca gag tat gca gca gtg cct ttc gat agc ttt act cta 483
Lys Leu Ala Thr Glu Tyr Ala Ala Val Pro Phe Asp Ser Phe Thr Leu
        140                 145                 150

cag aag gtg tac cag ctg gag act ggc ctt acc cgc cac ccc gag gag 531
Gln Lys Val Tyr Gln Leu Glu Thr Gly Leu Thr Arg His Pro Glu Glu
    155                 160                 165

aag cct gtg agg aag gac aag cgg gat gag ttg gtg gaa gcc att gaa 579
Lys Pro Val Arg Lys Asp Lys Arg Asp Glu Leu Val Glu Ala Ile Glu
170                 175                 180                 185

tca gcc ttg gag acc ctg aac aat cct gca gag aac agc ccc aat cac 627
Ser Ala Leu Glu Thr Leu Asn Asn Pro Ala Glu Asn Ser Pro Asn His
                190                 195                 200

cgt cct tac acg gcc tct gat ttc ata gaa ggg atc tac cga aca gaa 675
Arg Pro Tyr Thr Ala Ser Asp Phe Ile Glu Gly Ile Tyr Arg Thr Glu
            205                 210                 215

agg gac aaa ggg aca ttg tat gag ctc acc ttc aaa ggg gac cac aaa 723
Arg Asp Lys Gly Thr Leu Tyr Glu Leu Thr Phe Lys Gly Asp His Lys
        220                 225                 230

cac gaa ttc aaa cgg ctc atc tta ttt cga cca ttc agc ccc atc atg 771
His Glu Phe Lys Arg Leu Ile Leu Phe Arg Pro Phe Ser Pro Ile Met
    235                 240                 245

aaa gtg aaa aat gaa aag ctc aac atg gcc aac acg ctt atc aat gtt 819
Lys Val Lys Asn Glu Lys Leu Asn Met Ala Asn Thr Leu Ile Asn Val
250                 255                 260                 265

atc gtg cct cta gca aaa agg gtg gac aag ttc cgg cag ttc atg cag 867
Ile Val Pro Leu Ala Lys Arg Val Asp Lys Phe Arg Gln Phe Met Gln
                270                 275                 280

aat ttc agg gag atg tgc att gag cag gat ggg aga gtc cat ctc act 915
Asn Phe Arg Glu Met Cys Ile Glu Gln Asp Gly Arg Val His Leu Thr
            285                 290                 295

gtt gtt tac ttt ggg aaa gaa gaa ata aat gaa gtc aaa gga ata ctt 963
Val Val Tyr Phe Gly Lys Glu Glu Ile Asn Glu Val Lys Gly Ile Leu
        300                 305                 310

gaa aac act tcc aaa gct gcc aac ttc agg aac ttt acc ttc atc cag 1011

Glu Asn Thr Ser Lys Ala Ala Asn Phe Arg Asn Phe Thr Phe Ile Gln
    315                 320                 325

ctg aat gga gaa ttt tct cgg gga aag gga ctt gat gtt gga gcc cgc    1059
Leu Asn Gly Glu Phe Ser Arg Gly Lys Gly Leu Asp Val Gly Ala Arg
330                 335                 340                 345

ttc tgg aag gga agc aac gtc ctt ctc ttt ttc tgt gat gtg gac atc    1107
Phe Trp Lys Gly Ser Asn Val Leu Leu Phe Phe Cys Asp Val Asp Ile
                350                 355                 360

tac ttc aca tct gaa ttc ctc aat acg tgt agg ctg aat aca cag cca    1155
Tyr Phe Thr Ser Glu Phe Leu Asn Thr Cys Arg Leu Asn Thr Gln Pro
            365                 370                 375

ggg aag aag gta ttt tat cca gtt ctt ttc agt cag tac aat cct ggc    1203
Gly Lys Lys Val Phe Tyr Pro Val Leu Phe Ser Gln Tyr Asn Pro Gly
        380                 385                 390

ata ata tac ggc cac cat gat gca gtc cct ccc ttg gaa cag cag ctg    1251
Ile Ile Tyr Gly His His Asp Ala Val Pro Pro Leu Glu Gln Gln Leu
    395                 400                 405

gtc ata aag aag gaa act gga ttt tgg aga gac ttt gga ttt ggg atg    1299
Val Ile Lys Lys Glu Thr Gly Phe Trp Arg Asp Phe Gly Phe Gly Met
410                 415                 420                 425

acg tgt cag tat cgg tca gac ttc atc aat ata ggt ggg ttt gat ctg    1347
Thr Cys Gln Tyr Arg Ser Asp Phe Ile Asn Ile Gly Gly Phe Asp Leu
                430                 435                 440

gac atc aaa ggc tgg ggc gga gag gat gtg cac ctt tat cgc aag tat    1395
Asp Ile Lys Gly Trp Gly Gly Glu Asp Val His Leu Tyr Arg Lys Tyr
            445                 450                 455

ctc cac agc aac ctc ata gtg gta cgg acg cct gtg cga gga ctc ttc    1443
Leu His Ser Asn Leu Ile Val Val Arg Thr Pro Val Arg Gly Leu Phe
        460                 465                 470

cac ctc tgg cat gag aag cgc tgc atg gac gag ctg acc ccc gag cag    1491
His Leu Trp His Glu Lys Arg Cys Met Asp Glu Leu Thr Pro Glu Gln
    475                 480                 485

tac aag atg tgc atg cag tcc aag gcc atg aac gag gca tcc cac ggc    1539
Tyr Lys Met Cys Met Gln Ser Lys Ala Met Asn Glu Ala Ser His Gly
490                 495                 500                 505

cag ctg ggc atg ctg gtg ttc agg cac gag ata gag gct cac ctt cgc    1587
Gln Leu Gly Met Leu Val Phe Arg His Glu Ile Glu Ala His Leu Arg
                510                 515                 520

aaa cag aaa cag aag aca agt agc aaa aaa aca tga actcccagag         1633
Lys Gln Lys Gln Lys Thr Ser Ser Lys Lys Thr
            525                 530

aaggattgtg ggagacactt tttctttcct tttgcaatta ctgaaagtgg ctgcaacaga 1693

gaaaagactt ccataaagga cgacaaaaga attggactga tgggtcagag atg 1746

<210> 2
<211> 532
<212> PRT
<213> Homo sapiens

<400> 2
Met Met Met Val Arg Arg Gly Leu Leu Ala Trp Ile Ser Arg Val Val
1               5                   10                  15

Val Leu Leu Val Leu Leu Cys Cys Ala Ile Ser Val Leu Tyr Met Leu
            20                  25                  30

Ala Cys Thr Pro Lys Gly Asp Glu Glu Gln Leu Ala Leu Pro Arg Ala
        35                  40                  45

Asn Ser Pro Thr Gly Lys Glu Gly Tyr Gln Ala Val Leu Gln Glu Trp
    50                  55                  60

Glu Glu Gln His Arg Asn Tyr Val Ser Ser Leu Lys Arg Gln Ile Ala
65                  70                  75                  80

Gln Leu Lys Glu Glu Leu Gln Glu Arg Ser Glu Gln Leu Arg Asn Gly
                85                  90                  95

Gln Tyr Gln Ala Ser Asp Ala Ala Gly Leu Gly Leu Asp Arg Ser Pro
            100                 105                 110

Pro Glu Lys Thr Gln Ala Asp Leu Leu Ala Phe Leu His Ser Gln Val
        115                 120                 125

Asp Lys Ala Glu Val Asn Ala Gly Val Lys Leu Ala Thr Glu Tyr Ala
    130                 135                 140

Ala Val Pro Phe Asp Ser Phe Thr Leu Gln Lys Val Tyr Gln Leu Glu
145                 150                 155                 160

Thr Gly Leu Thr Arg His Pro Glu Glu Lys Pro Val Arg Lys Asp Lys
                165                 170                 175

Arg Asp Glu Leu Val Glu Ala Ile Glu Ser Ala Leu Glu Thr Leu Asn
            180                 185                 190

Asn Pro Ala Glu Asn Ser Pro Asn His Arg Pro Tyr Thr Ala Ser Asp
        195                 200                 205

Phe Ile Glu Gly Ile Tyr Arg Thr Glu Arg Asp Lys Gly Thr Leu Tyr
    210                 215                 220

Glu Leu Thr Phe Lys Gly Asp His Lys His Glu Phe Lys Arg Leu Ile
225                 230                 235                 240

Leu Phe Arg Pro Phe Ser Pro Ile Met Lys Val Lys Asn Glu Lys Leu
                245                 250                 255

Asn Met Ala Asn Thr Leu Ile Asn Val Ile Val Pro Leu Ala Lys Arg
            260                 265                 270

Val Asp Lys Phe Arg Gln Phe Met Gln Asn Phe Arg Glu Met Cys Ile
        275                 280                 285

Glu Gln Asp Gly Arg Val His Leu Thr Val Val Tyr Phe Gly Lys Glu
    290                 295                 300

Glu Ile Asn Glu Val Lys Gly Ile Leu Glu Asn Thr Ser Lys Ala Ala
305                 310                 315                 320

Asn Phe Arg Asn Phe Thr Phe Ile Gln Leu Asn Gly Glu Phe Ser Arg
                325                 330                 335

Gly Lys Gly Leu Asp Val Gly Ala Arg Phe Trp Lys Gly Ser Asn Val
            340                 345                 350

Leu Leu Phe Phe Cys Asp Val Asp Ile Tyr Phe Thr Ser Glu Phe Leu
        355                 360                 365

Asn Thr Cys Arg Leu Asn Thr Gln Pro Gly Lys Lys Val Phe Tyr Pro
    370                 375                 380

Val Leu Phe Ser Gln Tyr Asn Pro Gly Ile Ile Tyr Gly His His Asp
385                 390                 395                 400

Ala Val Pro Pro Leu Glu Gln Gln Leu Val Ile Lys Lys Glu Thr Gly
                405                 410                 415

Phe Trp Arg Asp Phe Gly Phe Gly Met Thr Cys Gln Tyr Arg Ser Asp
            420                 425                 430

Phe Ile Asn Ile Gly Gly Phe Asp Leu Asp Ile Lys Gly Trp Gly Gly
        435                 440                 445

Glu Asp Val His Leu Tyr Arg Lys Tyr Leu His Ser Asn Leu Ile Val
    450                 455                 460

Val Arg Thr Pro Val Arg Gly Leu Phe His Leu Trp His Glu Lys Arg
465                 470                 475                 480

Cys Met Asp Glu Leu Thr Pro Glu Gln Tyr Lys Met Cys Met Gln Ser
                485                 490                 495

Lys Ala Met Asn Glu Ala Ser His Gly Gln Leu Gly Met Leu Val Phe
            500                 505                 510

Arg His Glu Ile Glu Ala His Leu Arg Lys Gln Lys Gln Lys Thr Ser
        515                 520                 525

Ser Lys Lys Thr
    530

## Description

### Technical Field

The present invention relates to a sugar chain-cleaving agent which comprises a protein having activity of degrading chondroitin and chondroitin sulfate, and the like.

### Background of the Invention

In the following, the saccharides and saccharide residues to be described in the present invention represent D forms, unless otherwise indicated. In addition, the following abbreviations are used sometimes in this description.
N-Acetylgalactosamine: GaINAc
Glucuronic acid: GlcA
Chondroitin: CH
Chondroitin sulfate: CS
Chondroitin sulfate A: CSA
Chondroitin sulfate C: CSC
Chondroitin sulfate D: CSD
Chondroitin sulfate E: CDE
Dermatan sulfate: DS
Hyaluronic acid: HA
Heparan sulfate: HS
Heparin: Hep

Each of CH and CS is a kind of glycosaminoglycan having a basal backbone wherein a disaccharide in which GlcA is bound to GalNAc by a glycoside bond through β1,3 bond is repeatedly bound and continued through β1,4 glycoside bond. CSGalNAcT-1 is known as one of the enzymes which synthesize such CH and CS (*J. Biol. Chem.,* 277,38189-38196 (2002)).

CSGalNAcT-1 is known as an enzyme which has both of activity of transferring GalNAc to a specific tetrasaccharide structure (linkage tetrasaccharide) existing in the binding moiety with the protein (hereinafter also referred to as "initiation activity") and activity of transferring GalNAc to GlcA existing in the non-reducing end-group of the basal backbone of CH and CS (hereinafter also referred to as "elongation activity") in synthesizing CH and CS, but it is not known that such an enzyme has a function as an enzyme, so-called "chondroitinase", which degrades the basal backbone of CH and CS.

Human protein which function as chondroitinase have been hardly known so far. Also, for the purpose of applying it to a new medicament and the like, concern has been directed toward a sugar chain-cleaving agent which uses such an enzyme.

### Disclosure of the Invention

In order to solve the above-described problems, the present inventors have conducted intensive studies and found as a result that the conventionally known enzyme CSGa1NAcT-1 has activity of degrading the backbone of CH and CS, and the present invention was accomplished by applying it as a sugar chain degrading enzyme agent.

That is, the present invention relates to the followings.
(1) A sugar chain-cleaving agent (hereinafter referred to as "cleaving agent of the present invention"), which comprises the following protein (a) or (b) as an active ingredient:
   (a) a protein which comprises the amino acid sequence represented by amino acid numbers 37 to 532 in SEQ ID NO:2;
   (b) a protein which comprises an amino acid sequence having addition, deletion, substitution, insertion and/or transposition of one or several amino acids in the amino acid sequence represented by amino acid numbers 37 to 532 in SEQ ID NO:2, and has activity of degrading CH and/or CS.
      In this case, "CS" is preferably CSA and/or CSC. Also, CS is preferably derived from a whale cartilage and/or a shark cartilage. Furthermore, the protein to be used as an active ingredient of the cleaving agent of the present invention preferably consists of the amino acid sequence represented by amino acid numbers 37 to 532 in SEQ ID NO:2. In addition, the protein may form a glycoprotein bound to a sugar chain.
(2) Use of "a protein comprising the amino acid sequence represented by amino acid numbers 37 to 532 in SEQ ID NO:2 or a glycoprotein in which a sugar chain is bound to the protein" as a sugar chain-cleaving agent (hereinafter referred to as "use of the present invention").
(3) An agent which controls activity of the cleaving agent of the present invention (hereinafter referred to as "controlling agent of the present invention").
(4) A medicament which comprises the cleaving agent of the present invention as an active ingredient (hereinafter referred to as "medicament of the present invention").
   The medicament of the present invention is preferably an agent for treating a disease caused by change of activity of a CH degrading enzyme and/or a CS degrading enzyme. Also, the medicament of the present invention is preferably an agent for treating a disease caused by the presence of excess CH and/or CS. Furthermore, the medicament of the present invention is preferably used for specifically cleaving CH and/or CS. In addition, "CS" in these cases is preferably CSA and/or CSC.
(5) A method for specifically cleaving a sugar chain, which comprises reacting the following protein (a) or (b) with CH and/or CS (hereinafter referred to as "cleaving method of the present invention"):
   (a) a protein which comprises the amino acid sequence represented by amino acid numbers 37 to 532 in SEQ ID NO:2;
   (b) a protein which comprises an amino acid sequence having addition, deletion, substitution, insertion and/or transposition of one or several amino acids in the amino acid sequence represented by amino acid numbers 37 to 532 in SEQ ID NO:2, and has activity of degrading CH and/or CS.
      In this case, "CS" is preferably CSA and/or CSC. In addition, CS is preferably derived from a whale cartilage and/or a shark cartilage.
(6) A method for specifically producing a sugar chain of which molecular weight is decreased, which reacting the following protein (a) or (b) with CH and/or CS (hereinafter referred to as "production method of the present invention"):
   (a) a protein which comprises the amino acid sequence represented by amino acid numbers 37 to 532 in SEQ ID NO:2;
   (b) a protein which comprises an amino acid sequence having addition, deletion, substitution, insertion and/or transposition of one or several amino acids in the amino acid sequence represented by amino acid numbers 37 to 532 in SEQ ID NO:2, and has activity of degrading CH and/or CS.

In this case, "CS" is preferably CSA and/or CSC. In addition, CS is preferably derived from a whale cartilage and/or a shark cartilage.

The present invention is described below in detail based on the best mode for carrying out the invention.

### Brief Description of the Drawings

Fig. 1 shows separation of reaction products by gel filtration, formed by allowing CSGalNAcT-1 and CSGalNAcT-2 to react with various polysaccharides. a to e correspond to reaction products by CSGalNAcT-1, and f to j correspond to reaction products by CSGalNAcT-2. a and f show charts after treatment of CH, b and g show charts after treatment of CSA, c and h show charts after treatment of DS, d and i show charts after treatment of CSC, and e and j show charts after treatment of CSD.
Fig. 2 shows change of the construction of hexasaccharide, tetrasaccharide and disaccharide in the reaction product, with the elapse of time. Open circles correspond to hexasaccharide, closed circles correspond to tetrasaccharide and triangles correspond to disaccharide.
Fig. 3 shows radioactivity distribution in the reaction product at the time of digesting CH in which the non-reducing terminal was labeled with radioactivity, with CSGalNAcT-1. Open circles correspond to radioactivity distribution of ¹⁴C-GlcA, and closed circles correspond to radioactivity distribution of ³H-GalNAc.
Fig. 4 shows a result of MALDI-TOF-MS analysis of CH hexasaccharide prepared by digestion with hyaluronidase.
Fig. 5 shows a result of MALDI-TOF-MS analysis of CH hexasaccharide prepared by digestion with CSGalNAcT-1.
Fig. 6 shows a result of MALDI-TOF-MS analysis of CH tetrasaccharide prepared by digestion with hyaluronidase.
Fig. 7 shows a result of MALDI-TOF-MS analysis of CH tetrasaccharide prepared by digestion with CSGalNAcT-1.
Fig. 8 shows charts of digestion products prepared by digesting CH tetradecasaccharide and CSA tetradecasaccharide with CSGalNAcT-1, separated by HPLC. a corresponds to CH tetradecasaccharide, b corresponds to CSA tetradecasaccharide, c corresponds to digestion products of CH tetradecasaccharide digested with CSGalNAcT-1, d corresponds to digestion products of CS tetradecasaccharide digested with CSGalNAcT-1, and e corresponds to a chart of CSGalNAcT-1 alone.

### Best Mode for Carrying Out the Invention

### 1. Cleaving agent of the present invention, use of the present invention and controlling agent of the present invention

The cleaving agent of the present invention is a sugar chain-cleaving agent, which comprises the following protein (a) or (b) as an active ingredient:
(a) a protein which comprises the amino acid sequence represented by amino acid numbers 37 to 532 in SEQ ID NO:2;
(b) a protein which comprises an amino acid sequence having addition, deletion, substitution, insertion and/or transposition of one or several amino acids in the amino acid sequence represented by amino acid numbers 37 to 532 in SEQ ID NO:2, and has activity of degrading CH and/or CS.

The above-described protein of (a) as an active ingredient of the cleaving agent of the present invention is the same as the protein of the conventionally known CSGalNAcT-1 *(J. Biol Chem.,* 277, 38189-38196 (2002)).

In addition, it is known in general that enzyme activity is maintained when one or several (usually from 2 to 24) constituting amino acids in the amino acid sequence of an enzyme protein are added, deleted, substituted, inserted and/or transposed (hereinafter they are also simply referred to as "mutation" as a whole), and it can be said that a protein having such a mutation is a variant of the same enzyme. Also in the case of the cleaving agent of the present invention, even when the amino acid sequence of the above-described protein of (a) has a mutation of one or several constituting amino acids, it can be said that such a protein is substantially the same enzyme protein as the above-described protein of (a), so long as it has activity of degrading CH and/or CS. The above-described protein of (b) means such a protein. The "amino acid sequence having addition, deletion, substitution, insertion and/or transposition of one or several amino acids in the amino acid sequence represented by amino acid numbers 37 to 532 in SEQ ID NO:2" preferably has a homology of 95% or more (mutation of 24 or less amino acids), more preferably 96% or more (mutation of 19 or less amino acids), still more preferably 97% or more (mutation of 14 or less amino acids) and most preferably 98% or more (mutation of 10 or less amino acids), with the amino acid sequence represented by the amino acid numbers 37 to 532. Homology of amino acid sequences can be easily calculated using conventionally known computer software such as FASTA, and such software can be used for the application by internet.

As the above-described proteins of (a) and (b) which are the active ingredients of the cleaving agent of the present invention, the following proteins of (a') and (b') can be exemplified.
(a') a protein which consists of the amino acid sequence represented by amino acid numbers 37 to 532 in SEQ ID NO:2;
(b') a protein which consists of an amino acid sequence having addition, deletion, substitution, insertion and/or transposition of one or several amino acids in the amino acid sequence represented by amino acid numbers 37 to 532 in SEQ ID NO:2, and has activity of degrading CH and/or CS.

In the cleaving agent of the present invention, "CS" is preferably CSA and/or CSC, and CSC is particularly preferable. Also, CS is preferably derived from a whale cartilage and/or a shark cartilage.

In this connection, CSA is a CS which richly contains a chondroitin 4-sulfate structure, and CSC is a CS which richly contains a chondroitin 6-sulfate structure. Usually, CS derived from a whale cartilage is commercially available as CSA, and CS derived from a shark cartilage is commercially available as CSC.

Whether or not a protein has activity of degrading CH and/or CS can be judged, for example, by applying the products obtained by reacting the protein with CH and/or CS to a Superdex Peptide column (manufactured by Amersham Bioscience) or the like, separating them using 0.2 mol/l NaCl (flow rate 1 ml/min) or the like as the mobile phase, detecting the molecular weight distribution of sugar chains through the detection of an absorbance of ultraviolet region (e.g., 225 nm or the like is used), and comparing this with the absorbance of ultraviolet region of "standard CH oligosaccharide and/or CS oligosaccharide" prepared in accordance with the method described, for example, in *J. Biol. Chem.,* 277, 38179-38188 (2002).

In this connection, it is known that a region to which a sugar chain is bound, such as N-glycosyl region, is usually present in protein. Accordingly, the above-described protein as an active ingredient of the cleaving agent of the present invention may be in the form of "glycoprotein" in which a sugar chain is bound via such a sugar chain binding region. That is, not only the protein comprising the amino acid sequence represented by amino acid numbers 37 to 532 in SEQ ID NO:2, but a glycoprotein in which a sugar chain is bound to the protein can also be used as the sugar chain-cleaving agent.

The production method of the cleaving agent of the present invention is not particularly limited too, and the above-described protein of (a) or (b) may be isolated from a natural resource, the above-described protein of (a) or (b) may be produced by chemical synthesis or the like, or the above-described protein of (a) or (b) may be produced by genetic engineering techniques. The method for producing the cleaving agent of the present invention by genetic engineering techniques are explained in the cleaving agent production method of the present invention which is described later.

In addition, by the use of the above-described judging method, it becomes possible to screen a substance which can change (increase or decrease) the activity of the above-described protein of (a) or (b) to cleave CH and/or CS. The substance obtained by this screening can be used as the controlling agent of the present invention. Specifically, the controlling agent of the present invention can be obtained by a method for screening an activity controlling agent for the cleaving agent of the present invention, which comprises a step in which the activity of the cleaving agent of the present invention to cleave CH and/or CS is measured in the presence or absence of a candidate substance, whether or not the candidate substance can change activity of the cleaving agent of the present invention is judged thereby, and a candidate substance which changed the activity is selected as an activity controlling agent for the cleaving agent of the present invention.

### 2. Medicament of the present invention

The medicament of the present invention is a medicament which comprises the cleaving agent of the present invention as an active ingredient.

Regarding the medicament of the present invention, the disease as its application object is not particularly limited, so long as it uses the cleaving agent of the present invention (the above-described protein of (a) or (b)) as an active ingredient, and it is preferably an agent for treating a disease caused by the change of activities of a CH degrading enzyme and/or a CS degrading enzyme. This "change of activities" may be systemic or topical. Also, the "change of activities" is preferably "reduction of activities".

In addition, the medicament of the present invention may be an agent for treating a disease caused by the presence of excess CH and/or CS. The "excess" as used herein means a higher level than the level under healthy condition. Also, "the presence of excess CH and/or CS" may be systemic or topical. The disease caused by the presence of excess CH and/or CS includes, for example, disk herniation and the like.

Also, the medicament of the present invention is preferably used for specifically cleaving CH and/or CS. That is, the medicament of the present invention can also be applied to a disease or the like in which specific cleaving of CH and/or CS is desired. Such a disease includes, for example, disk herniation and the like.

"CS" in these cases is preferably CSA and/or CSC, and CSC is particularly preferable.

In addition, the term "treatment" as used in the present application documents includes all of the treatments for purposes such as prevention, progress inhibition (worsening prevention), improvement and treatment.

The moiety where the medicament of the present invention is administered is individually set, depending on the kind of diseases, onset region and the like, and is not particularly limited. For example, a living body tissue where CH and/or CS is excessively present can be cited. Such a living body tissue includes, for example, nucleus pulposus under a state of disk herniation. Accordingly, the medicament of the present invention can also be used as an agent for treating disk herniation.

In recent years, an attempt has been made to treat disk herniation by administering chondroitinase to intervertebral disk and thereby melting nucleus pulposus (US Patent No. 4,696,816, *Clinical Orthopaedics,* 253, 301-308 (1990)). Accordingly, the medicament of the present invention having activity of cleaving CH and CS can also be sufficiently used as an agent for treating disk herniation.

In addition, knowledge has been obtained in recent years that CH and CS inhibit regeneration of an injured nerve *(Nature,* 416, 6881, 589-590 (2002)), and regeneration of such an injured nerve can also be accelerated by administering the medicament of the present invention to the injured region of the nerve.

The animal species to which the medicament of the present invention is applied is also not particularly limited. When it is used as the above-described agent for treating disk herniation or nerve regeneration treating agent, the species is preferably a vertebrate, more preferably a mammal. The mammal includes, for example, human, dog, cat, rabbit, horse, sheep, monkey, bovine, pig, goat, mouse, rat and the like, and it is particularly preferable to apply it to human.

The administration method of the medicament of the present invention is not particularly limited, so long as the activity of the medicament of the present invention to cleave CH and CS is exerted, and administration by injection is exemplified. When the medicament of the present invention is applied to nucleus pulposus for example as a treating agent of disk herniation, it is preferable to inject it into intervertebral disk or spinal epidural cavity where the nucleus pulposus of interest is present.

In addition, the above-described protein of (a), (b), (a') or (b') can be suitably formulated to obtain the medicament of the present invention. Usually, the formulation is carried out by mixing it together with one or at least two pharmaceutically acceptable carriers according to any method well known in the technical field of manufacturing pharmacy. The dosage forms include injections (solutions, suspensions, emulsions, solid preparations for dissolution when used, and the like), tablets, capsules, solutions and the like. Among these, injections are particularly preferable. Dose of the medicament of the present invention is not particularly limited, because it should be individually decided, depending on the kind and specific activity of the above-described protein, animal species to be administered, kind and symptom the disease, kind and conditions of the living body tissue as the object of administration and the like, and usually about from 0.1 µg to 1000 mg per day can be administered.

In addition, the medicament of the present invention may contain other components, so long as it contains the cleaving agent of the present invention as an active ingredient. Such "other components" are not particularly limited, so long as they do not substantially exert bad influences upon the cleaving agent of the present invention which is the active ingredient of the medicament of the present invention, and are pharmaceutically acceptable. Accordingly, not only the pharmaceutically acceptable carriers and the like, but also other physiologically active components and the like can be used as the "other components".

Description on the "cleaving agent of the present invention" as an active ingredient of the medicament of the present invention is the same as described above. The method for producing the medicament of the present invention by genetic engineering techniques is described in "5. Production method of the cleaving agent of the present invention" which is described later.

### 3. Cleaving method of the present invention

The cleaving method of the present invention is a method for specifically cleaving the sugar chain, which comprises reacting the following protein (a) or (b) with CH and/or CS:
(a) a protein which comprises the amino acid sequence represented by amino acid numbers 37 to 532 in SEQ ID NO:2;
(b) a protein which comprises an amino acid sequence having addition, deletion, substitution, insertion and/or transposition of one or several amino acids in the amino acid sequence represented by amino acid numbers 37 to 532 in SEQ ID NO:2, and has activity of degrading CH and/or CS.

The above-described proteins of (a) and (b) are as described above.

The method for reacting such a protein with CH and/or CS is not particularly limited, so long as these molecules mutually contact to cause conditions under which an enzyme reaction is generated, and for example, the latter may be allowed to contact with the former, the former may be allowed to contact with the latter, or both of them may be simultaneously allowed to contact. For example, CH and/or CS may be allowed to contact with a carrier (e.g., gel, beads, membrane, plate or the like) prepared by fixing the above-described protein of (a) or (b) thereto. By the use of such a carrier, both of them can also be contacted continuously.

The reaction conditions in carrying out the contact of both of them are not particularly limited, so long as they are conditions under which the above-described proteins can function, but it is preferable to allow these proteins to react at around their optimum pH (e.g., pH of approximately from 5 to 7), and it is more preferable to carry out the reaction in a buffer having the buffer action under the pH. The temperature in this case is also not particularly limited, so long as the activities of these proteins are maintained, and approximately from 35 to 40°C is preferable.

In addition, when a substance which increases activities of these proteins is available, the substance may be added. The reaction time can be optionally set, depending on the pH conditions, temperature conditions, amount of the protein and amount of the sugar chain to be used in the reaction, desired degree of cleaving (reduction of molecular weight) and the like. For example, the degree of cleaving (reduction of molecular weight) can be increased when the reaction time is prolonged, and the degree can be decreased when the reaction time is shortened.

In addition, the cleaving method of the present invention comprises such a protein reaction step, and it may further comprise other step.

By the cleaving method of the present invention, CH and/or CS is specifically cleaved. In the cleaving agent of the present invention, "CS" is preferably CSA and/or CSC, and CSC is particularly preferable. Also, CS is preferably derived from a whale cartilage and/or a shark cartilage.

Whether or not these sugar chains were cleaved can be detected by a usual method for detecting the sugar chain of which molecular weight is decreased. This method is as described above. The cleaving method of the present invention is preferably a method which can specifically cleave only CH and CS.

In addition, the cleaving method of the present invention is preferably a method which does not cleave DS, CSD, CSE, HA, N-acetylheparosan, HS and Hep. Un-cleaving of these the sugar chains can also be confirmed by the usual method for detecting a sugar chain of which molecular weight is decreased.

That is, the cleaving method of the present invention is preferably used for cleaving CH, CSA and CSC (particularly CH and CSC) and used for not cleaving DS, CSD, CSE, HA, N-acetylheparosan, HS and Hep.

Other descriptions regarding the cleaving method of the present invention are the same as the above-described the cleaving agent of the present invention.

### 4. Production method of the present invention

The production method of the present invention is a method for specifically producing a sugar chain of which molecular weight is decreased, which comprises reacting the following protein (a) or (b) with CH and/or CS:
(a) a protein which comprises the amino acid sequence represented by amino acid numbers 37 to 532 in SEQ ID NO:2;
(b) a protein which comprises an amino acid sequence having addition, deletion, substitution, insertion and/or transposition of one or several amino acids in the amino acid sequence represented by amino acid numbers 37 to 532 in SEQ ID NO:2, and has activity of degrading CH and/or CS.

In this case, "CS" is preferably CSA and/or CSC, and CSC is particularly preferable. In addition, CS is preferably derived from a whale cartilage and/or a shark cartilage.

By the production method of the present invention, a sugar chain in which the molecular weight of CH and/or CS is decreased can be specifically produced. Descriptions of the production method of the present invention are the same as the case of the above-described cleaving method of the present invention.

Accordingly, particularly, the production method of the present invention is preferably a method which produces only CH, CSA and CSC (particularly CH and CSC) and does not produce DS, CSD, CSE, HA, N-acetylheparosan, HS and Hep. Production of such sugar chains or no production of the same can also be confirmed by the usual method for detecting a sugar chain of which molecular weight is decreased.

According to the production method of the present invention, it further comprises collecting a sugar chain of which molecular weight is decreased, after reacting the protein of (a) or (b) with CH and/or CS. The method for collecting and isolating the sugar chain of which molecular weight is decreased and the like can be carried out by conventionally known methods.

### 5. Cleaving agent production method of the present invention

The cleaving agent production method of the present invention is a production method of the cleaving agent of the present invention, which comprises expressing a protein using a DNA containing either of the following (c) or (d), and collecting the expressed protein:
(c) a DNA encoding a protein which comprises the amino acid sequence represented by amino acid numbers 37 to 532 in SEQ ID NO: 2;
(d) a DNA encoding a protein which comprises an amino acid sequence having addition, deletion, substitution, insertion and/or transposition of one or several amino acids in the amino acid sequence represented by amino acid numbers 37 to 532 in SEQ ID NO:2, and has activity of degrading CH and/or CS.

The above-described DNA of (c) is not particularly limited, so long as it encodes a protein which comprises the amino acid sequence represented by amino acid numbers 37 to 532 in SEQ ID NO:2. As such a DNA, various DNA molecules having different nucleotide sequences due to degeneracy of genetic code are present, but a DNA specified by nucleotide numbers of 25 to 1623 in the nucleotide sequence represented by SEQ ID NO:1 is preferable, and a DNA specified by nucleotide numbers of 133 to 1623 in the nucleotide sequence represented by SEQ ID NO:1 is more preferable.

The above-describe DNA of (d) is not particularly limited too, so long as it encodes a protein which comprises an amino acid sequence having addition, deletion, substitution, insertion and/or transposition of one or several amino acids in the amino acid sequence represented by amino acid numbers 37 to 532 in SEQ ID NO:2, and has activity of degrading CH and/or CS. Such a DNA includes, for example, a DNA which hybridizes with the DNA described in the above-described (c), a DNA complementary to the DNA or DNA having the nucleotide sequence of such a DNA, under stringent conditions.

In this connection, the "stringent conditions" mean conditions under which so-called specific hybrid is formed but nonspecific hybrid is not formed (cf. Sambrook, J*. et al., Molecular Cloning, A Laboratory Manual,* Second Edition, Cold Spring Harbor Laboratory Press (1989) and the like). The "stringent conditions" specifically include conditions in which hybridization is carried out at 42°C in a solution containing 50% formamide, 4 x SSC, 50 mM HEPES (pH 7.0), 10 x Denhardt's solution and 100 µg/ml of salmon sperm DNA, followed by washing with 2 x SSC, 0.1% SDS solution at room temperature and with 0.1 x SSC, 0.1% SDS solution at 50°C.

A protein using a DNA retaining either of the above-described (c) or (d) is preferably expressed using a vector (preferably an expression vector) which retains the DNA. The DNA can be inserted into a vector by a usual method.

As the vector into which the DNA is inserted, for example, an appropriate expression vector (phage vector, plasmid vector or the like) capable of expressing the inserted DNA can be used, and it can be suitably selected according to the host cell to be introduced with the vector of the present invention. Such a host-vector system includes, for example, a combination of a mammalian cell such as COS cell or 3LL-HK46 cell with an expression vector for mammalian cell such as pGIR201 (Kitagawa, H and Paulson, J.C., *J. Biol. Chem.,* 269, 1394-1401 (1994)), pEF-BOS (Mizushima, S. and Nagata, S, *Nucleic Acid Res.,* 18, 5322 (1990)), pCXN2 (Niwa, H., Yamanura, K. and Miyazaki, J., *Gene,* 108, 193-200 (1991)), pCMV-2 (manufactured by Eastman Kodak), pCEV18, pME18S (Maruyama *et al., Med Immunol.,* 20, 27 (1990)) or pSVL (manufactured by Pharmacia Biotech); a combination of *Escherichia coli (E. coli)* with an expression vector for procaryotic cell such as pTrcHis (manufactured by Invitrogen), pGEX (manufactured by Pharmacia Biotech), pTrc99 (manufactured by Pharmacia Biotech), pKK233-3 (manufactured by Pharmacia Biotech), pEZZZ18 (manufactured by Pharmacia Biotech), pCH110 (manufactured by Pharmacia Biotech), pET (manufactured by Stratagene), pBAD (manufactured by Invitrogen), pRSET (manufactured by Invitrogen) or pSE420 (manufactured by Invitrogen); and a combination of an insect cell with a baculovirus, as well as yeast, *Bacillus subtilis* or the like as a host cell and various vectors corresponding thereto. Between the above-described host-vector systems, a combination of a mammalian cell (particularly COS cell) with pFLAG-CMV6 (manufactured by SIGMA) and a combination of an insect cell with a baculovirus are particularly preferable.

In addition, as the vector to be inserted with the DNA, those which are constructed for the purpose of expressing a fusion protein of the protein of interest with a marker peptide can also be used. The protein can be expressed from the DNA and the expressed protein can be collected in accordance with usual methods.

For example, this can be carried out by introducing an expression vector inserted with the DNA of interest into an appropriate host for transformation in the host, growing this transformant, and then collecting the expressed protein from the grown matter.

The "growth" as used herein is a general idea which includes propagation of a cell or the microorganism itself as a transformant and growth of an animal, insect or the like into which the cell as the transformant is introduced. Also, the "grown matter" as used herein is a general idea which includes a medium after growing of the transformant (supernatant of the cultured medium), and cultured host cell, secreted substance, excreted substance and the like. Conditions for the growth (medium, culturing conditions and the like) can be suitably selected according to the host to be used.

The protein can be collected from the grown matter by conventionally known methods for extracting and purifying protein.

For example, when the intended protein is produced in a soluble form which is secreted into a medium (supernatant of the cultured medium), the medium may be collected and used as such. Also, when the intended protein is produced in a soluble form which is secreted into the cytoplasm or in an insoluble (membrane binding) form, the intended protein can be extracted by extraction through a cell disruption means such as a method using a nitrogen cavitation device, homogenization, a glass beads method, ultrasonic treatment, an osmotic pressure shock method or a freeze-thawing method; extraction with a surfactant; a combination thereof; or the like, and the extract may be used as such.

The protein can be further purified from these medium and extract. The purification may be imperfect purification (partial purification) or perfect purification, which can be suitably selected according to the using object and the like of the protein of interest.

The specific purification method includes, for example, salting out by ammonium sulfate, sodium sulfate or the like, centrifugation, dialysis, ultrafiltration, adsorption chromatography, ion exchange chromatography, hydrophobic chromatography, reverse phase chromatography, gel filtration method, gel permeation chromatography, affinity chromatography, electrophoresis and the like: a combination thereof; and the like.

Whether or not the protein of interest is produced can be confirmed by analyzing its amino acid sequence, actions, substrate specificity and the like.

Also, the cleaving agent production method of the present invention comprises at least the expression and collection steps as described above, and may further comprise other steps.

In addition, the medicament of the present invention can also be produced by using the cleaving agent of the present invention obtained in this manner as an active ingredient.

The present invention is further described below in more detail based on Example, but the present invention is not limited thereto.

### Example

A fusion protein of an estimated catalytic domain of human derived CSGalNAcT-1 (the amino acid sequence represented by amino acid numbers 37 to 532 in SEQ ID NO: 2) with FLAG peptide was produced by genetic engineering techniques in accordance with the method described in *J. Biol. Chem.,* 277, 38189-38196 (2002). This fusion protein is a protein in the solubilized form. This fusion protein was adhered to agarose gel to which an anti-FLAG antibody M2 (manufactured by Sigma) was bound. This agarose gel was washed with 50 mmol/l Tris buffer (pH 7.4) containing 20% glycerol, and then the supernatant was removed by suction. This gel was suspended in 50 mmol/l MES buffer (pH 6.5; containing 10 mmol/l of MnCl₂ and 17.1 mmol/l of adenosine triphosphate (ATP), and glycosaminoglycan (CH (prepared by removing sulfate from whale cartilage derived CSA with acidic methanol solution; manufactured by Seikagaku Corporation), CSA (derived from whale cartilage; manufactured by Seikagaku Corporation), DS (derived from pig skin; manufactured by Seikagaku Corporation), CSC (derived from shark cartilage; manufactured by Seikagaku Corporation), CSD (derived from shark cartilage; manufactured by Seikagaku Corporation), CSE (derived from squid cartilage; manufactured by Seikagaku Corporation), HA (derived from cockscomb; manufactured by Seikagaku Corporation), N-acetylheparosan (derived from capsular polysaccharide of *E. coli* K5; manufactured by Seikagaku Corporation), HS (derived from pig aorta; manufactured by Seikagaku Corporation) or Hep (derived from pig small intestines; manufactured by SIGMA)) to a concentration of 0.2 mg/ml and used as the reaction liquid.

After 50 µl of the reaction liquid was stirred on a shaker at 37°C for 60 minutes, the reaction was then stopped by denaturing the protein through boiling of the reaction liquid at 100°C for 5 minutes, and the supernatant was passed through a 0.22 µm filter to obtain a sample. The sample was applied to a Superdex Peptide column (manufactured by Pharmacia: 0.2 mol/l of NaCl was used as the mobile phase, and its flow rate was set to 1 ml/min) and detected at an absorbance of 225 nm. CH oligosaccharide to be used as the molecular weight marker was prepared in accordance with the method of *J. Biol. Chem.,* 277, 38179-38188 (2002). In addition, the human derived CSGalNAcT-1 was changed to human derived CSGalNAcT-2 and used as a negative control. The CSGalNAcT-2 was prepared in accordance with *J. Biol. Chem.,* 278, 38189-38196 (2002).

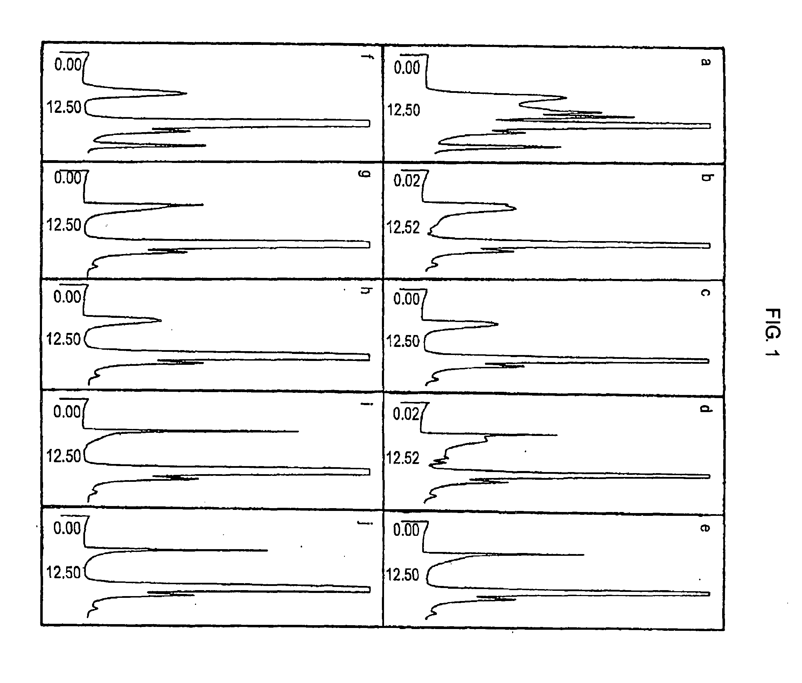

When CSGalNAcT-1 was allowed to react with CH, CSA, DS, CSC, CSD, (results are not shown on the following) CSE, HS, N-acetylheparosan, HS or Hep, decrease of the polysaccharide molecular weight was observed in CH and CSC, and slight decrease of the polysaccharide molecular weight was observed in CSA (Fig. 1). Decrease of the molecular weight was not observed in the negative control. Based on this result, it was shown that CSGa1NAcT-1 has activity of decreasing the molecular weight of polysaccharide. Also, it was suggested that CSGa1NAcT-1 recognizes the disaccharide repeating structure of CH, and the sulfate group exerts influence upon the activity.

Also, as a cause of the only slight decrease of the molecular weight observed in CSA, influence of GalNAc in which the 4-position was sulfated (GalNAc-4S: about 60% of the constituting GalNAc residues), which is present in the used CSA, was suggested. In addition, since there is no inhibition when chelating of divalent metal ions is effected by adding ethylenediaminetetraacetic acid (EDTA) to the reaction system, it is considered that the above-described activity does not require divalent metals.

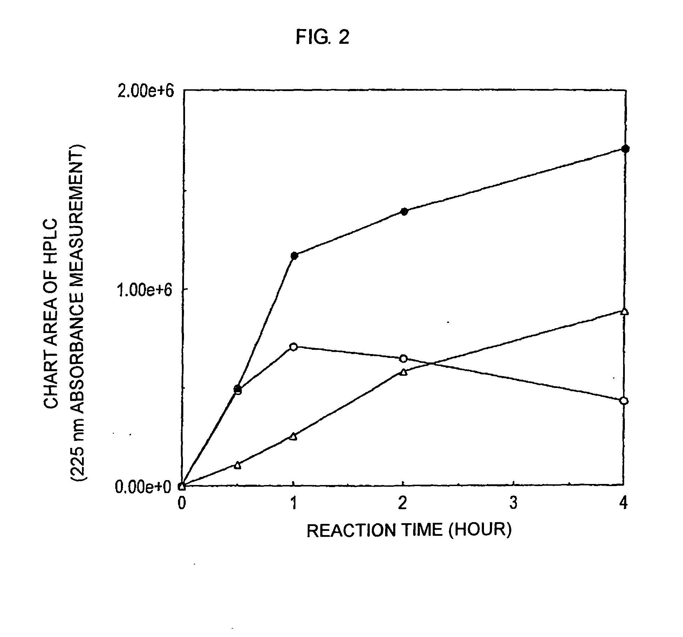

When this degradation product was further fractionated by a Superdex Peptide column (manufactured by Pharmacia), the degradation products are considered to be hexasaccharide, tetrasaccharide and disaccharide based on their elution positions, and it was found that hexasaccharide is firstly formed and then tetrasaccharide and disaccharide are increased, from the change of elution patterns of the degradation product degraded by changing the reaction time (Fig. 2). Based on this, it was suggested that the activity of CSGalNAcT-1 decreasing the molecular weight of CH/CS polysaccharide is an endo type.

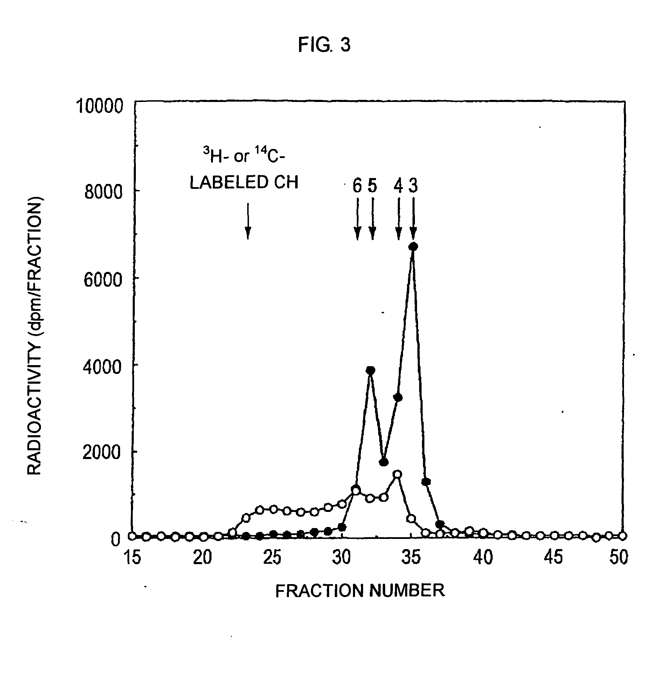

Next, CH in which its non-reducing tarminal was labeled with [¹⁴C]GlcA or [³H]GalNAc by a usual method using CSS3 (*J. Biol. Chem.,* 278, 39711-39725 (2003)) was enzymatically digested with CSGalNAcT-1 and fractionated using a Superdex Peptide column (manufactured by Pharmacia), and then radioactivity of each fraction was measured by a scintillation counter. As a result, radioactivity peaks of the degradation products of the substance labeled with [¹⁴C]GlcA were detected at the elution positions of hexasaccharide and tetrasaccharide, and those of the substance labeled with [³H]GalNAc at the elution positions of pentasaccharide and trisaccharide (Fig. 3). Based on this, it was suggested that the GalNAc·1-4GlcA bond was cleaved by CSGalNAcT-1, at least regarding specificity of the non-reducing end-group side oligosaccharide. In addition, since the radioactivity was not detected at the elution position of disaccharide, it was shown that the hexasaccharide or pentasaccharide is firstly cleaved, and then the non-reducing terminal side of large fragments are selectively degraded.

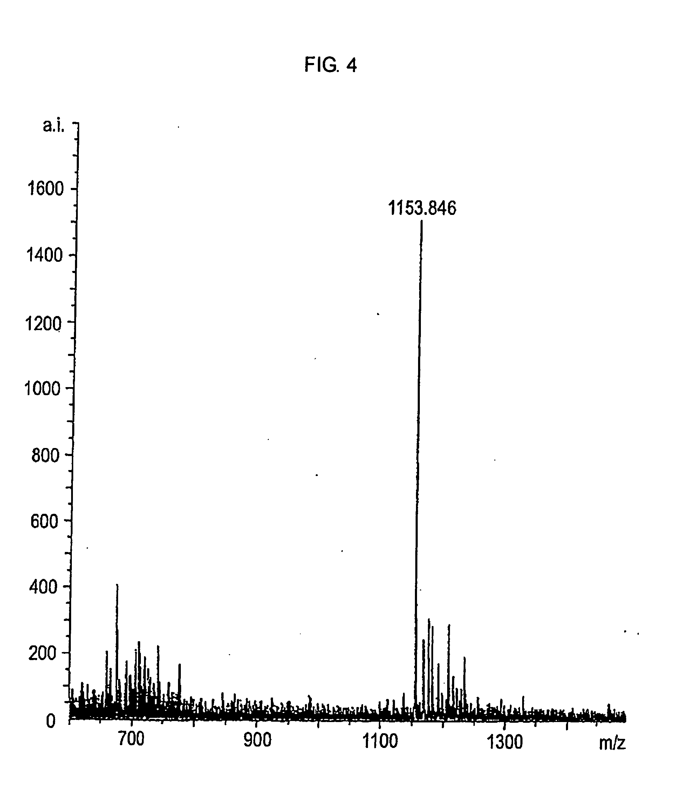

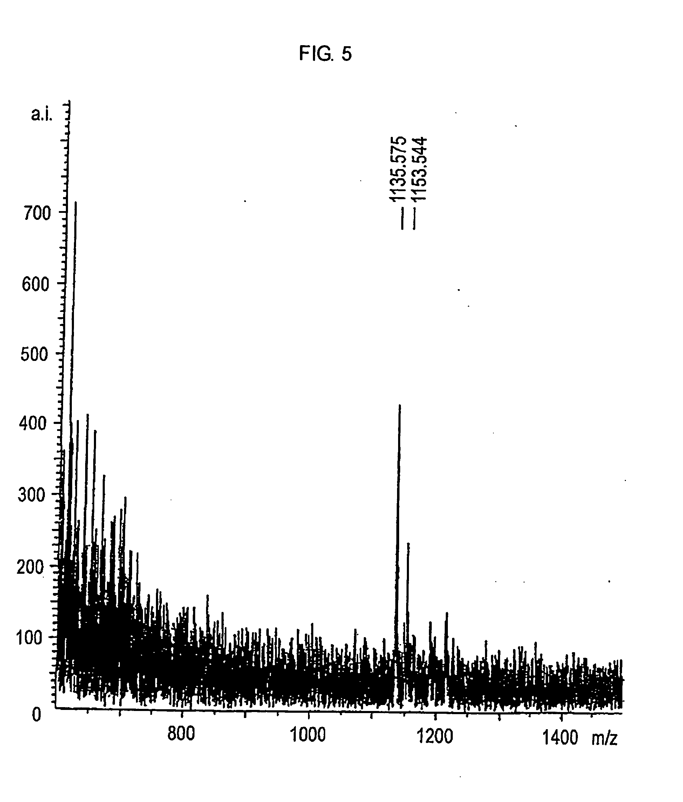

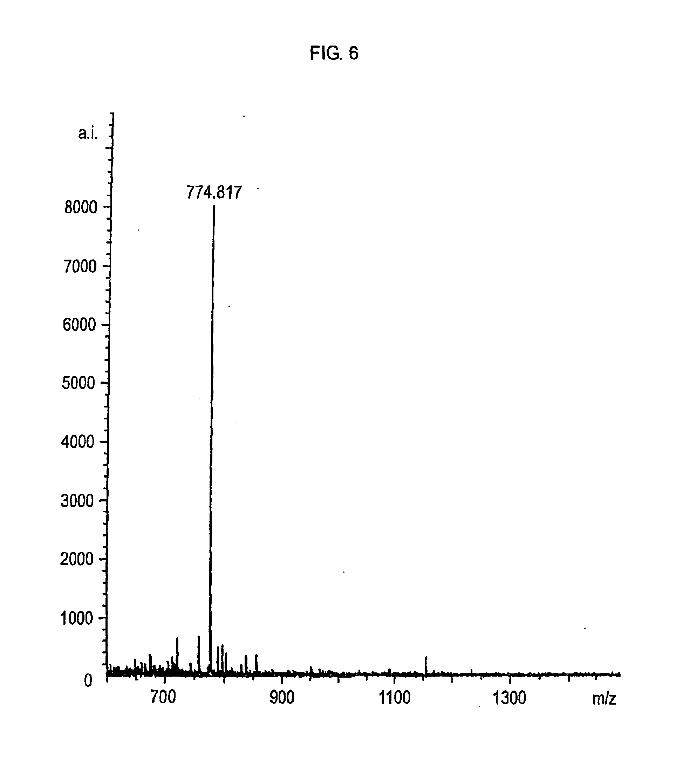

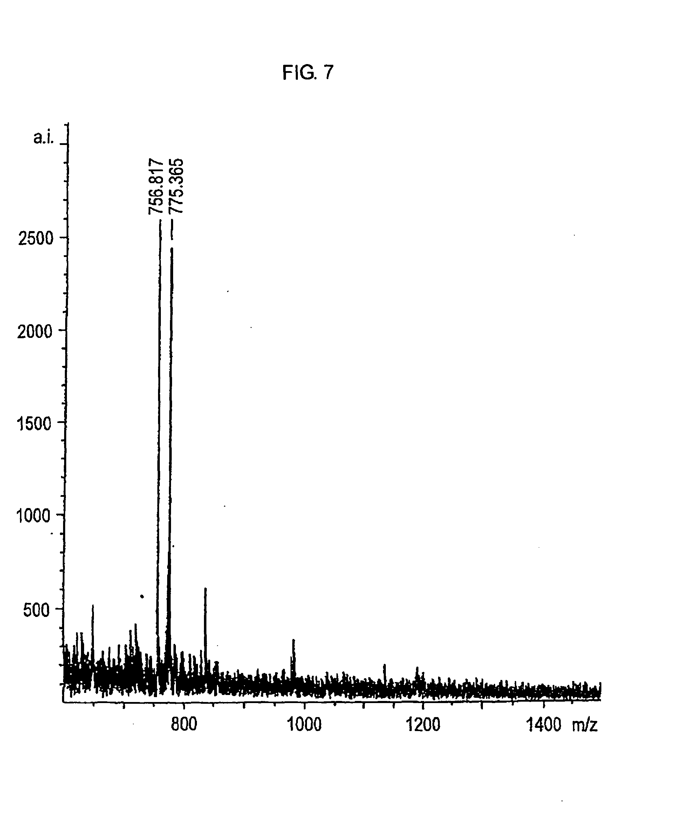

When the hexasaccharide and tetrasaccharide formed by reacting CSGalNAcT-1 with CH were purified by the method described in *J. Biol. Chem., 277*, 38179-38188 (2002) and analyzed by matrix-assisted laser desorption ionization-time-of-flight mass spectrometry (MALDI-TOF-MS: Reflex IV (manufactured by Brucker Daltonics)), peaks of the hexasaccharide and tetrasaccharide smaller by a factor of 18 Da than those of the hexasaccharide and tetrasaccharide (molecular weight markers) obtained by digesting CH with hyaluronidase were detected (hexasaccharide: Fig. 4 and Fig. 5, tetradecasaccharide: Fig. 6 and Fig. 7). Based on this result, a possibility was suggested that this enzyme activity is an eliminase such as endo--N-acetylgalactosaminidase or cell-derived chondroitinase.

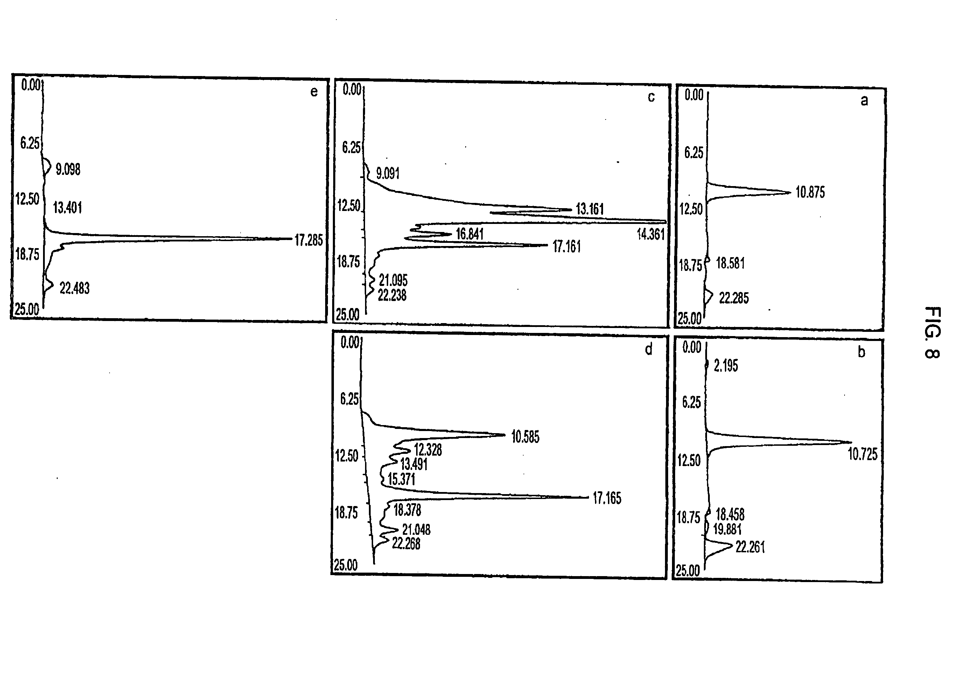

Since this activity was not observed when CSA, DS or CSD was used as the substrate, it is considered that it is influenced by sulfation of the substrate, so that a possibility was suggested that the GalNAc-4S structure commonly possessed by CSA, DS and CSD acts in an inhibitive manner. Accordingly, the activity was measured using chondroitin sulfate tetradecasaccharide (the 4-sulfate group is contained in almost all of its 7 GalNAc residues), prepared in accordance with *Biochem. J.,* 226, 705-714 (1985) from a sturgeon notochord derived chondroitin sulfate A sample (manufactured by Seikagaku Corporation) which contains the GalNAc-4S structure at a high frequency of 92.5%, or chondroitin tetradecasaccharide (manufactured by Seikagaku Corporation) as the substrate, to find that the substrate was hardly degraded in the case of the use of the chondroitin sulfate tetradecasaccharide (Fig. 8). Based on this, it was shown that the GalNAc-4S structure at least contained in chondroitin sulfate acts upon this activity in an inhibitory manner.

While the present invention has been described in detail and with reference to specific embodiments thereof, it will be apparent to one skilled in the art that various changes and modifications can be made therein without departing from the spirit and scope of the present invention.

This application is based on a Japanese patent application filed on August 26, 2003 (Japanese Patent Application No. 2003-301943), entire contents thereof being thereby incorporated by reference. All references cited herein are incorporated in their entirety.

### Industrial Applicability

According to the present invention, a protein having ability of degrading CH and CS is provided, and a sugar chain-cleaving agent which specifically degrades CH and CS sulfate and the like are provided by using the same.

## Claims

1. A sugar chain-cleaving agent, which comprises the following protein (a) or (b) as an active ingredient:
(a) a protein which comprises the amino acid sequence represented by amino acid numbers 37 to 532 in SEQ ID NO: 2;
(b) a protein which comprises an amino acid sequence having addition, deletion, substitution, insertion and/or transposition of one or several amino acids in the amino acid sequence represented by amino acid numbers 37 to 532 in SEQ ID NO:2, and has activity of degrading chondroitin and/or chondroitin sulfate.

2. The sugar chain-cleaving agent according to claim 1, wherein the chondroitin sulfate is chondroitin sulfate A and/or chondroitin sulfate C.

3. The sugar chain-cleaving agent according to claim 1 or 2, wherein the chondroitin sulfate is chondroitin sulfate derived from a whale cartilage and/or chondroitin sulfate derived from a shark cartilage.

4. The sugar chain-cleaving agent according to any one of claims 1 to 3, wherein the protein consists of the amino acid sequence represented by amino acid numbers 37 to 532 in SEQ ID NO:2

5. The sugar chain-cleaving agent according to any one of claims 1 to 4, wherein the protein is a glycoprotein bound to a sugar chain.

6. Use of a protein comprising the amino acid sequence represented by amino acid numbers 37 to 532 in SEQ ID NO:2 or a glycoprotein in which a sugar chain is bound to the protein as a sugar chain-cleaving agent.

7. An agent which controls activity of the sugar chain-cleaving agent according to any one of claims 1 to 5.

8. A medicament which comprises the sugar chain-cleaving agent as an active ingredient according to any one of claims 1 to 5.

9. The medicament according to claim 8, which is an agent for treating a disease caused by change of activity of a chondroitin degrading enzyme and/or a chondroitin sulfate degrading enzyme.

10. The medicament according to claim 8, which is an agent for treating a disease caused by the presence of excess chondroitin and/or chondroitin sulfate.

11. The medicament according to any one of claims 8 to 10, which is used for the purpose of specifically cleaving chondroitin and/or chondroitin sulfate.

12. The medicament according to any one of claims 9 to 11, wherein the chondroitin sulfate is chondroitin sulfate A and/or chondroitin sulfate C.

13. A method for specifically cleaving a sugar chain, which comprises reacting the following protein (a) or (b) with chondroitin and/or chondroitin sulfate:
(a) a protein which comprises the amino acid sequence represented by amino acid numbers 37 to 532 in SEQ ID NO:2;
(b) a protein which comprises an amino acid sequence having addition, deletion, substitution, insertion and/or transposition of one or several amino acids in the amino acid sequence represented by amino acid numbers 37 to 532 in SEQ ID NO:2, and has activity of degrading chondroitin and/or chondroitin sulfate.

14. The method according to claim 13, wherein the chondroitin sulfate is chondroitin sulfate A and/or chondroitin sulfate C.

15. The method according to claim 13 or 14, wherein the chondroitin sulfate is chondroitin sulfate derived from a whale cartilage and/or chondroitin sulfate derived from a shark cartilage.

16. A method for specifically producing a sugar chain of which molecular weight is decreased, which reacting the following protein (a) or (b) with chondroitin and/or chondroitin sulfate:
(a) a protein which comprises the amino acid sequence represented by amino acid numbers 37 to 532 in SEQ ID NO:2;
(b) a protein which comprises an amino acid sequence having addition, deletion, substitution, insertion and/or transposition of one or several amino acids in the amino acid sequence represented by amino acid numbers 37 to 532 in SEQ ID NO:2, and has activity of degrading chondroitin and/or chondroitin sulfate.

17. The method according to claim 16, wherein the chondroitin sulfate is chondroitin sulfate A and/or chondroitin sulfate C.

18. The method according to claim 15 or 17, wherein the chondroitin sulfate is chondroitin sulfate derived from a whale cartilage and/or chondroitin sulfate derived from a shark cartilage.
